# FASCICULE DE BREVET EUROPEEN

(11) **EP 0 679 087 B1**
(45) Date de publication et mention de la délivrance du brevet: **02.04.1997**
(21) Numéro de dépôt: 94904666.8
(22) Date de dépôt: 12.01.1994
(51) Int. Cl.: A61K 35/78, A61K 7/48

(54) **COMPOSITION COSMETIQUE OU PHARMACEUTIQUE, NOTAMMENT DERMATOLOGIQUE, CONTENANT UN EXTRAIT DE VISMIA**
VISMIA EXTRAKT ENTHALTENDE KOSMETISCHE ODER PHARMAZEUTISCHE ZUBEREITUNG INSBESONDERS DERMATOLOGISCH.
COSMETIC OR PHARMACEUTICAL AND PARTICULARLY DERMATOLOGICAL COMPOSITION CONTAINING A VISMIA EXTRACT

(30) Priorité: 13.01.1993 FR 9300263
(43) Date de publication de la demande: 02.11.1995
(73) Titulaire: LVMH RECHERCHE, 92752 Nanterre (FR)
(72) Inventeur: BONTE, Frédéric, F-92400 Courbevoie (FR); MEYBECK, Alain Les Poissons, F-92400 Courbevoie (FR)
(74) Mandataire: Portal, Gérard
(86) Numéro de dépôt international: FR9400036
(87) Numéro de publication internationale: WO9415626

(56) Documents cités:
- BOTANICAL MUSEUM LEAFLETS vol. 29, no. 1 , 1983 pages 49 - 57 R. E. SCHULTES 'DE PLANTIS TOXICARIIS E MUNDO NOVO TROPICALE COMMENTATIONES XXX'
- PHYTOCHEMISTRY vol. 25, no. 5 , 1986 pages 1217 - 1219 B. BOTTA ET AL. 'VISMIONE H AND PRENYLATED XANTHONES FROM VISMIA GUINEENSIS'

## Description

La présente invention concerne essentiellement une composition cosmétique ou pharmaceutique, notamment dermatologique, contenant un extrait de Vismia.

Plus précisément, la présente invention concerne l'utilisation d'un extrait de Vismia, pour la préparation d'une composition cosmétique ou pharmaceutique, notamment dermatologique, stimulant notamment la synthèse du collagène, et destinée en particulier à lutter contre les effets du vieillissement cutané, à obtenir un raffermissement cutané, à améliorer la cicatrisation ou à traiter les diverses pathologies accompagnées d'une déficience en collagène, ainsi que des compositions cosmétiques ou pharmaceutiques en comportant application.

Les plantes du genre Vismia sont connues de longue date dans le bassin amazonien et les Guyanes pour leur utilisation médicinale, pour soigner les dermatoses ainsi que des éruptions cutanées, ou encore comme laque ou cire à cacheter.

On connaît par ailleurs par le document Journal of Natural Products, 1986, volume 49, n° 5, pages 929-931, l'activité antitumorale des vismiones isolées de Vismia.

Il a maintenant été découvert une activité surprenante des extraits de Vismia sur la synthèse de collagène, en particulier de collagène de type I, ci-après dénommé en abréviation "collagène I", ce qui les rend particulièrement utiles pour lutter contre les effets du vieillissement cutané, ainsi que pour obtenir un raffermissement cutané, ou améliorer la cicatrisation.

La présente invention a donc pour but principal de résoudre le nouveau problème technique consistant en la fourniture d'une nouvelle formulation d'une composition cosmétique ou pharmaceutique, notamment dermatologique, présentant une bonne efficacité sur la prévention ou le traitement des effets du vieillissement cutané, ainsi que sur le raffermissement cutané, ou améliorer la cicatrisation.

La présente invention a encore pour but principal de résoudre ce nouveau problème technique d'une manière particulièrement simple, ne nécessitant pas obligatoirement l'isolement d'une substance active particulière ou sa synthèse, satisfaisante et utilisable à l'échelle industrielle, notamment dans l'industrie cosmétique ou pharmaceutique.

Ainsi, selon un premier aspect, la présente invention concerne l'utilisation d'un extrait de Vismia, pour la préparation d'une composition cosmétique ou pharmaceutique, notamment dermatologique, stimulant notamment la synthèse du collagène, en particulier celle du collagène I, et destinée en particulier à lutter contre les effets du vieillissement cutané, à obtenir un raffermissement cutané ou à traiter les diverses pathologies accompagnées d'une déficience en collagène.

Suivant une variante de réalisation la composition précitée est destinée à améliorer la cicatrisation cutanée.

Selon une variante de réalisation préférée, l'extrait précité est obtenu à partir d'une plante du genre Vismia choisie parmi le groupe consistant de Vismia cayennensis, Vismia guianensis (Aublet), Vismia macrophylla Kunth, Vismia latifolia (Aublet), Vismia sandwithii Ewans, Vismia sessilifolia, Vismia guineensis, Vismia angusta, Vismia confertiflora, Vismia amazonica, Vismia dealbata, Vismia ferruginea, Vismia tomentosa, Vismia baccifera, Vismia lindeniama, Vismia japurensis ; en particulier à partir des écorces ou des fruits de cette plante.

Selon une variante de réalisation, l'extrait précité est obtenu à partir d'une plante du genre Vismia cayennensis, Vismia amazonica ou Vismia guianensis.

Selon une variante de réalisation particulière, l'extrait de Vismia précité est obtenu par extraction par un solvant polaire, tel que le méthanol ou un mélange hydro-éthanolique, de préférence à partir de l'écorce ou des fruits de la plante.

En particulier, l'extrait de Vismia peut être obtenu selon le procédé décrit ci-après à titre indicatif, mais nullement limitatif.

On effectue une première extraction de l'écorce ou des fruits de la plante, par un solvant polaire, avantageusement choisi parmi le groupe constitué par : l'eau, les alcools comportant de préférence de 1 à 4 atomes de carbone, les solvants chlorés comportant de préférence 1 à 2 atomes de carbone, les esters organiques comportant de préférence de 3 à 6 atomes de carbone ; ou d'un solvant mixte à base d'un mélange quelconque des solvants précités.

Encore de préférence, le solvant de première extraction est choisi parmi le groupe consistant de l'eau, le méthanol, l'éthanol, un mélange méthanol-eau ou un mélange éthanol-eau, le chloroforme, le dichlorométhane. Encore de préférence, il s'agit de l'eau, du méthanol, de l'éthanol ou de leurs mélanges.

Le rapport de l'écorce ou des fruits à l'agent d'extraction n'est pas critique et généralement sera compris entre 1 : 5 et 1 : 20 parties en poids.

L'extraction s'effectue généralement à des températures comprises entre la température ambiante et le point d'ébullition du solvant utilisé pour l'extraction.

De préférence, cette première extraction est effectuée au reflux sous pression atmosphérique pendant une durée de 2 à 4 h. En outre, elle est avantageusement précédée d'une macération à froid pendant 2 à 4 h dans le solvant d'extraction.

En fin d'extraction, la phase du solvant contenant l'extrait est filtrée puis concentrée et/ou évaporée à sec sous pression réduite. On obtient ainsi un premier extrait brut de Vismia selon l'invention. Cet extrait brut peut être purifié selon divers procédés bien connus à l'homme de l'art.

Selon un second aspect, la présente invention concerne aussi une composition cosmétique caractérisée en ce qu'elle comprend, à titre d'ingrédient actif, une quantité cosmétiquement efficace d'un extrait de Vismia.

Selon une variante de réalisation particulière, cette composition cosmétique stimulant la synthèse du collagène, en particulier celle du collagène I, est destinée notamment à lutter contre les effets du vieillissement cutané, ou à obtenir un raffermissement cutané. Une telle composition, par exemple, peut avantageusement être utilisée comme composition pour prévenir l'apparition des rides ou en atténuer la profondeur.

Selon un troisième aspect, l'invention concerne encore une composition pharmaceutique, notamment dermatologique, stimulant la synthèse du collagène, en particulier celle du collagène I, caractérisée en ce qu'elle comprend, à titre d'ingrédient actif, une quantité pharmaceutiquement efficace d'un extrait de Vismia.

Selon un mode de réalisation particulier, ladite composition est destinée à améliorer la cicatrisation ou à traiter les diverses pathologies accompagnées d'une déficience en collagène.

Dans l'un ou l'autre des aspects précédents, on utilisera de préférence l'extrait de Vismia à une concentration comprise entre 0,0001 % et 1 % en poids par rapport au poids total de la composition finale. Ceci est particulièrement le cas lorsque l'extrait est incorporé dans un excipient, véhicule ou support cosmétiquement ou pharmaceutiquement acceptable pour constituer une composition cosmétique ou pharmaceutique.

D'autres buts, caractéristiques et avantages de l'invention apparaîtront clairement à la lecture de la description explicative qui va suivre, faite en référence à plusieurs exemples donnés uniquement à titre d'illustration et qui ne sauraient par conséquent en aucune façon limiter la portée de l'invention.

Dans les exemples, les pourcentages sont exprimés en poids, sauf indication contraire. Dans le cas des extraits, les quantités de ceux-ci sont exprimées en poids sec.

### Exemple 1

### Préparation d'un extrait méthanolique d'écorce de Vismia cayennensis

On prend des écorces de Vismia cayennensis séchées que l'on broie finement. Puis 100 g du broyat est repris dans 900 ml de méthanol. Le tout est porté à ébullition et maintenu à cette température pendant 1 h, puis on filtre pour éliminer les écorces. On réalise ensuite une évaporation du solvant, par exemple dans un évaporateur rotatoire sous pression réduite et on récupère un extrait concentré dit extrait I₁.

### Exemple 2

### Préparation d'un extrait hydro-éthanolique de fruits de Vismia amazonica

On procède comme décrit à l'exemple 1 si ce n'est que l'on utilise des fruits de Vismia amazonica et que le solvant est unmélange eau-éthanol 30/70. On peut répéter l'extraction à chaud jusqu'à épuisement.

On regroupe les différents extraits et on réalise l'évaporation comme décrit à l'exemple 1 jusqu'à obtention d'un extrait sec dit extrait I₂.

### Exemple 3

### Mise en évidence de l'activité d'un extrait méthanolique d'écorce de Vismia cayennensis préparé selon l'exemple 1, extrait I₁, sur la synthèse de collagène par des fibroblastes humains

On effectue un prélèvement de fibrobaste sur la peau d'une femme blanche de 60 ans au cours d'un lifting et on coupe la peau en fines bandelettes que l'on place dans une solution de trypsine à 0,25 % pendant une nuit à 4°C. Les cellules épidermiques se détachent alors du derme après grattage.

Les bandelettes de derme ainsi obtenues sont découpées en fragments cubiques d'environ 1 mm de côté, nommés explants. Ces explants sont déposés sur un film de milieu E 199 GIBCO®, supplémenté avec 2 mM de L-glutamine et 10 % de sérum de voeu foetal, à raison de 20 explants par boîte de culture de 100 mm de diamètre.

Les boîtes sont incubées à 37°C et l'atmosphère humide enrichie en CO₂ (5 %). Le milieu est renouvelé deux fois par semaine.

Lorsque les cellules atteignent la confluence autour des explants, les fibroblastes sont extraits sans prélever les explants, par l'emploi d'un mélange trypsine 0,1 %-EDTA, 0,02 %, et les fibroblastes sont réimplantés dans un milieu de culture renouvelé. Certaines boîtes servent de témoins et reçoivent du milieu renouvelé sans le produit de l'invention, les autres boîtes sont divisées en deux lots recevant respectivement le produit de l'invention à 10 µg/ml.

On laisse incuber pendant 48 h en l'absence ou en présence du produit de l'invention à la concentration indiquée.

Après 48 h d'incubation, on procède à un dosage immunoenzymatique du collagène I sécrété par les fibroblastes dans le surnageant, par une méthode similaire à celle décrite par RENNARD S.I. et al dans Anal. Biochem. (1980), 104, 205-214.

Les résultats obtenus par cette méthode de dosage sont répertoriés au tableau I ci-après.

La significativité des résultats est déterminée au moyen du test de Student, avec p < 0,05.

**TABLEAU I**

| Cultures | Collagène I sécrété par fibroblastes (ng/10000 cellules/48 h) | % de stimulation | Significativité |
|---|---|---|---|
| Culture témoin sans produit | 635,2 ± 29,9 | 0 | |

| Produit de l'invention I₁ | | | |
|---|---|---|---|
| 10 µg/ml | 1 306,4 ± 38,4 | + 105,7 | S* |

| | | | |
|---|---|---|---|
| *S = significatif | | | |

Au tableau I, on a exprimé les quantités de collagène I sécrétées par les fibroblastes en ng/10000 cellules/48 h.

On observera, à partir du tableau I, que l'extrait méthanolique de Vismia cayennensis a produit une activité significative de stimulation de la synthèse de collagène I par les fibroblastes.

A ce sujet, Shuster et al., dans Br. J. Dermatol. (1975), 93, 639-643, ayant pour titre "The Influence of age, and sex on skin thickness, skin collagen and density", ont mis en évidence une diminution du contenu en collagène de la peau entre 15 et 93 ans. De plus, le collagène est à la base de l'architecture du derme, d'où l'intérêt de produit capable d'exercer un effet de stimulation de la synthèse de collagène I.

### Exemple 4

### Mise en évidence de l'activité d'un extrait hydro-éthanolique de fruits de Vismia amazonica, préparé selon l'exemple 2, extrait I₂, sur la synthèse du collagène par des fibroblastes humains

Selon la méthodologie décrite à l'exemple 3, on utilise l'extrait de fruits de Vismia amazonica obtenu à l'exemple 2, extrait I₂, en lieu et place de l'extrait d'écorces de Vismia cayennensis obtenu à l'exemple 1, tandis que le prélèvement de fibroblastes a été réalisé sur la peau d'une femme blanche de 56 ans également au cours d'un lifting, et ce, à diverses concentrations non cytotoxiques d'extrait mentionnées au tableau II ci-après.

La significativité est déterminée, comme dans l'exemple précédent, au moyen du test de Student, avec p < 0,05.

**TABLEAU II**

| Cultures | Collagène I sécrété par fibroblastes (ng/10000 cellules/48 h) | % de stimulation | Significativité |
|---|---|---|---|
| Culture témoin sans produit | 430 ± 40 | 0 | |

| Produit de l'invention I₂ | | | |
|---|---|---|---|
| 1,25 µg/ml | 508 ± 60 | +18 | S |
| 5 µg/ml | 543 ± 56 | +26 | S |
| 12,5 µg/ml | 540 ± 59 | +26 | S |

On peut ainsi constater, à partir du tableau II, que les extraits par un mélange hydro-éthanolique de fruits de Vismia produisent également une activité significative sur la stimulation de la synthèse de collagène I par les fibroblastes.

Ainsi, les extraits de Vismia peuvent avantageusement être utilisés, grâce à leur propriété de stimulation de la synthèse du collagène, comme agent actif, dans des compositions cosmétiques ou pharmaceutiques, notamment dermatologiques, telles que définies précédemment.

Diverses formulations de compositions cosmétiques sont données ci-après :

### Exemple 5

### Gel de massage raffermissant cutané

| | |
|---|---|
| - Extrait d'écorce de Vismia selon l'invention de l'exemple 1 | 0,1 g |
| - Ethanol | 25 g |
| - Glycérine | 2 g |
| - Propylèneglycol | 2 g |
| - Carbopol 940® | 1,25 g |
| - Excipient aqueux avec conservateur éventuellement parfumé q.s.p. | 100,00 g |

Une partie de l'excipient aqueux est utilisée avec le Carbopol pour préparer séparément un gel, l'autre partie de l'excipient aqueux est utilisée pour être mélangée avec les autres composants et le gel est ajouté dans la solution obtenue afin d'obtenir une composition gélifiée formant le gel de massage.

Cette composition de gel de massage peut être utilisée trois fois par semaine pendant deux mois au niveau du buste.

### Exemple 6

### Lotion de soins du corps pour le raffermissement cutané

| | |
|---|---|
| - Extrait de fruit de Vismia selon l'exemple 2 | 0,5 g |
| - Agent solubilisant (Cremophor RH40®) | 2 g |
| - Acide hyaluronique | 1 g |
| - Excipient aqueux contenant un conservateur éventuellement parfumé q.s.p. | 100,00 g |

L'extrait est tout d'abord solubilise dans l'agent solubilisant, puis est ajouté dans l'excipient aqueux auquel on ajoute l'acide hyaluronique.

La lotion obtenue peut être utilisée par cure de trois semaines sur les zones sensibles au relâchement, telles que le ventre, les cuisses.

### Exemple 7

### Emulsion anti-ride

| | |
|---|---|
| - Extrait d'écorce de Vismia cayennensis selon l'exemple 1 | 0,10 g |
| - Excipient émulsionné parfumé q.s.p. | 100,00 g |

### Exemple 8

### Composition cicatrisante

| | |
|---|---|
| - Extrait d'écorce de Vismia cayennensis selon l'exemple 1 | 0,5 g |
| - Excipient émulsionné, type eau dans l'huile q.s.p. | 100,00 g |

## Revendications

1. Utilisation d'un extrait de Vismia pour la préparation d'une composition pharmaceutique, notamment dermatologique, stimulant la synthèse du collagène, en particulier celle du collagène I, et destiné en particulier à lutter contre les effets du vieillissement cutané, à obtenir un raffermissement cutané ou à traiter les diverses pathologies accompagnées d'une déficience en collagène.

2. Utilisation d'un extrait de Vismia comme agent cosmétique, notamment pour lutter contre les effets du vieillissement cutané, pour prévenir l'apparition des rides ou en atténuer la profondeur, ou pour obtenir un raffermissement cutané.

3. Utilisation selon la revendication 1 ou 2, caractérisée en ce que l'extrait précité est obtenu à partir d'une plante du genre Vismia choisie parmi le groupe consistant de Vismia cayennensis, Vismia guianensis (Aublet), Vismia macrophylla Kunth, Vismia latifolia (Aublet), Vismia sandwithii Ewans, Vismia sessilifolia, Vismia guineensis, Vismia angusta, Vismia confertiflora, Vismia amazonica, Vismia dealbata, Vismia ferruginea, Vismia tomentosa, Vismia baccifera, Vismia lindeniama, Vismia japurensis ; en particulier à partir des écorces ou des fruits de cette plante.

4. Utilisation selon l'une des revendications 1 à 3, caractérisée en ce que l'extrait précité est obtenu à partir de Vismia cayennensis, de Vismia amazonica ou de Vismia guianensis.

5. Utilisation selon l'une des revendications 1 à 4, caractérisée en ce que l'extrait de Vismia précité est obtenu par extraction par un solvant polaire, tel que le méthanol ou un mélange hydro-éthanolique, de préférence à partir de l'écorce ou des fruits de la plante.

6. Composition cosmétique, caractérisée en ce qu'elle comprend, à titre d'ingrédient actif, une quantité cosmétiquement efficace d'un extrait de Vismia.

7. Composition cosmétique selon la revendication 6, caractérisée en ce qu'il s'agit d'une composition cosmétique stimulant la synthèse du collagène, en particulier celle du collagène I, et destinée notamment à lutter contre les effets du vieillissement cutané, à prévenir l'apparition des rides ou en atténuer la profondeur, ou à obtenir un raffermissement cutané.

8. Composition pharmaceutique, notamment dermatologique, stimulant la synthèse du collagène, en particulier celle du collagène I, caractérisée en ce qu'elle comprend, à titre d'ingrédient actif, une quantité pharmaceutiquement efficace d'un extrait de Vismia.

9. Composition selon la revendication 8, caractérisée en ce que ladite composition pharmaceutique est destinée à améliorer la cicatrisation ou à traiter les diverses pathologies accompagnées d'une déficience en collagène.

10. Composition selon l'une des revendications 6 à 9, caractérisée en ce que la concentration en extrait de Vismia est comprise entre 0,0001 % et 1 % en poids par rapport au poids total de la composition finale.

11. Composition selon l'une des revendications 6 à 10, caractérisée en ce que l'extrait précité est obtenu à partir d'une plante du genre Vismia choisie parmi le groupe consistant de Vismia cayennensis, Vismia guianensis (Aublet), Vismia macrophylla Kunth, Vismia latifolia (Aublet), Vismia sandwithii Ewans, Vismia sessilifolia, Vismia guineensis, Vismia angusta, Vismia confertiflora, Vismia amazonica, Vismia dealbata, Vismia ferruginea, Vismia tomentosa, Vismia baccifera, Vismia lindeniama, Vismia japurensis ; en particulier à partir des écorces ou des fruits de cette plante.

12. Composition selon l'une des revendications 6 à 11, caractérisée en ce que l'extrait précité est obtenu à partir de Vismia cayennensis, de Vismia amazonica, ou de Vismia guianensis.

13. Composition selon l'une des revendications 6 à 12, caractérisée en ce que l'extrait de Vismia précité est obtenu par extraction par un solvant polaire, tel que le méthanol ou un mélange hydro-éthanolique, de préférence à partir de l'écorce ou des fruits de la plante.

14. Composition selon l'une des revendications 6 à 13, caractérisée en ce que l'extrait de Vismia précité est choisi parmi un extrait méthanolique d'écorce de Vismia cayennensis et un extrait hydro-éthanolique de fruit de Vismia amazonica.

15. Procédé de traitement cosmétique, caractérisé en ce qu'il comprend l'application topique sur une zone du corps d'une quantité cosmétiquement efficace d'un extrait de Vismia.

## Claims

1. Use of a Vismia extract for the preparation of a pharmaceutical, in particular dermatological composition which stimulates, collagen synthesis, especially that of collagen I, and is intended especially for combating the effects of skin aging, for obtaining a firming of the skin or for treating the various pathologies accompanied by a collagen deficiency.

2. Use of a Vismia extract as a cosmetic agent, in particular for combating the effects of skin aging, for preventing the appearance of wrinkles or smoothing them out or for obtaining a firming of the skin.

3. Use according to Claim 1 or 2, characterized in that the abovementioned extract is obtained from a plant of the genus Vismia chosen from the group consisting of Vismia cayennensis, Vismia guianensis (Aublet), Vismia macrophylla Kunth, Vismia latifolia (Aublet), Vismia sandwithii Ewans, Vismia sessilifolia, Vismia guineensis, Vismia angusta, Vismia confertiflora, Vismia amazonica, Vismia dealbata, Vismia ferruginea, Vismia tomentosa, Vismia baccifera, Vismia lindeniama and Vismia japurensis; especially from the barks or fruits of this plant.

4. Use according to one of Claims 1 to 3, characterized in that the abovementioned extract is obtained from Vismia cayennensis, from Vismia amazonica or from Vismia guianensis.

5. Use according to one of Claims 1 to 4, characterized in that the abovementioned Vismia extract is obtained by extraction with a polar solvent such as methanol or an aqueous-ethanolic mixture, preferably from the bark or fruits of the plant.

6. Cosmetic composition, characterized in that it comprises as active ingredient a cosmetically effective amount of a Vismia extract.

7. Cosmetic composition according to Claim 6, characterized in that it is a cosmetic composition which stimulates collagen synthesis, especially that of collagen I, and is intended, in particular, for combating the effects of skin aging, for preventing the appearance of wrinkles or smoothing them out or for obtaining a firming of the skin.

8. Pharmaceutical, in particular dermatological composition which stimulates collagen synthesis, especially that of collagen I, characterized in that it comprises as active ingredient a pharmaceutically effective amount of a Vismia extract.

9. Composition according to Claim 8, characterized in that the said pharmaceutical composition is intended for improving cicatrization or for treating the various pathologies accompanied by a collagen deficiency.

10. Composition according to one of Claims 6 to 9, characterized in that the concentration of Vismia extract is between 0.0001% and 1% by weight relative to the total weight of the final composition.

11. Composition according to one of Claims 6 to 10, characterized in that the abovementioned extract is obtained from a plant of the genus Vismia chosen from the group consisting of Vismia cayennensis, Vismia guianensis (Aublet), Vismia macrophylla Kunth, Vismia latifolia (Aublet), Vismia sandwithii Ewans, Vismia sessilifolia, Vismia guineensis, Vismia angusta, Vismia confertiflora, Vismia amazonica, Vismia dealbata, Vismia ferruginea, Vismia tomentosa, Vismia baccifera, Vismia lindeniama and Vismia japurensis; especially from the barks or fruits of this plant.

12. Composition according to one of Claims 6 to 11, characterized in that the abovementioned extract is obtained from Vismia cayennensis, from Vismia amazonica or from Vismia guianensis.

13. Composition according to one of Claims 6 to 12, characterized in that the abovementioned Vismia extract is obtained by extraction with a polar solvent such as methanol or an aqueous-ethanolic mixture, preferably from the bark or fruits of the plant.

14. Composition according to one of Claims 6 to 13, characterized in that the abovementioned Vismia extract is chosen from a methanolic extract of Vismia cayennensis bark and an aqueous-ethanolic extract of Vismia amazonica fruit.

15. Cosmetic treatment process, characterized in that it comprises the topical application to an area of the body of a cosmetically effective amount of a Vismia extract.

## Patentansprüche

1. Verwendung eines Vismia-Extrakts zur Herstellung einer pharmazeutischen, insbesondere dermatologischen, Zusammensetzung zur Stimulation der Synthese des Collagens, insbesondere des Collagens I, der insbesondere dazu bestimmt ist, die Effekte der Hautalterung zu bekämpfen, eine Straffung der Haut zu erreichen oder die verschiedenen Krankheitsbilder zu behandeln, die auf einen Collagenmangel zurückzuführen sind.

2. Verwendung eines Vismia-Extrakts als kosmetisches Mittel, insbesondere zur Bekämpfung der Effekte der Hautalterung, um die Faltenbildung zu verhindern oder die Faltentiefe zu mildern oder um eine Hautstraffung zu erreichen.

3. Verwendung nach den Ansprüchen 1 oder 2, dadurch gekennzeichnet, daß der vorgenannte Extrakt aus einer Pflanze der Vismia-Familie gewonnen wird, die aus jener Gruppe ausgewählt wird, die aus Vismia cayennensis, Vismia guianensis (Aublet), Vismia macrophylla Kunth, Vismia latifolia (Aublet), Vismia sandwithii Ewans, Vismia sessililfolia, Vismia guineensis, Vismia angusta, Vismia confertiflora, Vismia amazonica, Vismia dealbata, Vismia ferruginea, Vismia tomentosa, Vismia baccifera, Vismia lindeniama, Vismia japurensis bestehen, wobei der Extrakt insbesondere aus den Rinden oder den Früchten dieser Pflanzen gewonnen wird.

4. Verwendung nach einem der Ansprüche 1 bis 3, dadurch gekennzeichnet, daß der vorgenannte Extrakt aus der Vismia cayennensis, der Vismia amazonica oder der Vismia guianensis gewonnen wird.

5. Verwendung nach einem der Ansprüche 1 bis 4, dadurch gekennzeichnet, daß der genannte Vismia-Extrakt durch Extraktion durch ein polares Lösungsmittel, wie Methanol oder eine Hydro-Ethanol-Mischung, gewonnen wird, vorzugsweise aus der Rinde oder den Früchten der Pflanze.

6. Kosmetische Zusammensetzung, dadurch gekennzeichnet, daß sie als aktiven Bestandteil eine kosmetisch wirksame Menge eines Vismia-Extrakts enthält.

7. Kosmetische Zusammensetzung nach Anspruch 6, dadurch gekennzeichnet, daß es sich um eine kosmetische Zusammensetzung handelt, die die Synthese des Collagens, insbesondere die des Collagens I, stimuliert und insbesondere dazu bestimmt ist, die Effekte der Hautalterung zu bekämpfen, die Faltenbildung zu verhindern oder die Faltentiefe zu mildern oder eine Hautstraffung zu erreichen.

8. Pharmazeutische, insbesondere dermatologische, Zusammensetzung, zur Stimulierung der Synthese des Collagens, insbesondere jener des Collagens I, dadurch gekennzeichnet, daß sie als aktiven Bestandteil eine pharmazeutisch wirksame Menge eines Vismia-Extrakts enthält.

9. Zusmamensetzung nach Anspruch 8, dadurch gekennzeichnet, daß die genannte phamazeutische Zusammensetzung dazu bestimmt ist, die Vernarbung zu verbessern oder verschiedene Krankheitsbilder zu behandeln, die auf einen Collagenmangel zurückzuführen sind.

10. Zusammensetzung nach einem der Ansprüche 6 bis 9, dadurch gekennzeichnet, daß die Konzentration an Vismia-Extrakt zwischen 0,0001 und 1 Gew.-% bezogen auf das Gesamtgewicht der Endzusammensetzung liegt.

11. Zusammensetzung nach einem der Ansprüche 6 bis 10, dadurch gekennzeichnet, daß der vorgenannte Extrakt aus einer Pflanze der Vismia-Familie gewonnen wird, die aus jener Gruppe ausgewählt wird, die aus Vismia cayennensis, Vismia guianensis (Aublet), Vismia macrophylla Kunth, Vismia latifolia (Aublet), Vismia sandwithii Ewans, Vismia sessililfolia, Vismia guineensis, Vismia angusta, Vismia confertiflora, Vismia amazonica, Vismia dealbata, Vismia ferruginea, Vismia tomentosa, Vismia baccifera, Vismia lindeniama, Vismia japurensis bestehen, wobei der Extrakt insbesondere aus den Rinden oder den Früchten dieser Pflanzen gewonnen wird.

12. Zusammensetzung nach einem der Ansprüche 6 bis 11, dadurch gekennzeichnet, daß der vorgenannte Extrakt aus der Vismia cayennensis, der Vismia amazonica oder der Vismia guianensis gewonnen wird.

13. Zusammensetzung nach einem der Ansprüche 6 bis 12, dadurch gekennzeichnet, daß der genannte Vismia-Extrakt durch Extraktion durch ein polares Lösungsmittel, wie Methanol oder eine Hydro-Ethanol-Mischung, gewonnen wird, vorzugsweise aus der Rinde oder den Früchten der Pflanze.

14. Zusammensetzung nach einem der Ansprüche 6 bis 13, dadurch gekennzeichnet, daß der vorgenannte Vismia-Extrakt aus einem Methanolextrakt der Rinde der Vismia cayennensis und einem Hydro-Ethanolextrakt der Frucht der Vismia amazonica ausgewählt wird.

15. Verfahren zur kosmetischen Behandlung, dadurch gekennzeichnet, daß auf die Oberfläche eines Körperteiles eine kosmetisch wirksame Menge eines Vismia-Extrakts aufgetragen wird.
